Europäisches Patentamt
European Patent Office
Office européen des brevets

(19)

(11) Publication number: **0 091 185**
**A2**

(12) # EUROPEAN PATENT APPLICATION

(21) Application number: 83300920.2

(22) Date of filing: 22.02.83

(51) Int. Cl.³: C 07 D 455/03
A 61 K 31/435

(30) Priority: 06.04.82 HU 105382

(43) Date of publication of application:
12.10.83 Bulletin 83/41

(84) Designated Contracting States:
AT BE CH DE FR GB IT LI NL SE

(71) Applicant: CHINOIN Gyogyszer és Vegyészeti Termékek Gyára RT
To utca 1-5
H-1045 Budapest IV(HU)

(72) Inventor: Szantay, Csaba
Zsombolyai u 8
H-1113 Budapest(HU)

(72) Inventor: Szabo, Lajos
Visegradi u 38/b
H-1132 Budapest(HU)

(72) Inventor: Toth, Istvan
Bele Kiraly u 16
H-1125 Budapest(HU)

(72) Inventor: Szekeres, Laszlo
Kazinczy u 2
H-6720 Szeged(HU)

(72) Inventor: Papp, Gyula
Becsi Krt 37-39
H-6722 Szeged(HU)

(72) Inventor: Udvardy, Eva
Olajos u 2/A
H-6723 Szeged(HU)

(72) Inventor: Virag, Sandor
Sallai I u 29/a
H-1136 Budapest(HU)

(74) Representative: Skailes, Humphrey John et al,
Frank B. Dehn & Co. Imperial House 15-19 Kingsway
London WC2B 6UZ(GB)

(54) **Berbane derivatives.**

(57) Pharmaceutical compositions comprising racemic or optically active berbane derivatives of the general Formula VI

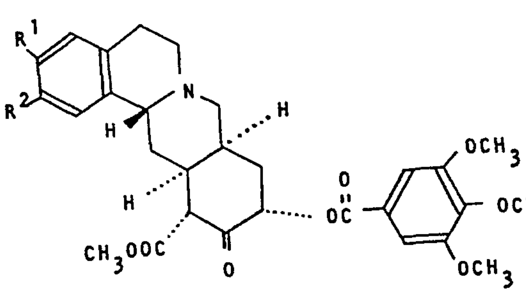

(VI)

wherein

$R^1$ and $R^2$ each stands for an alkyl group having 1-4 carbon atoms or

$R^1$ and $R^2$ together form a methylenedioxy group and pharmaceutically acceptable salts thereof.

These derivatives have hypotensive activity, and the compounds in which $R^1$ and $R^2$ are $C_{2-4}$ alkyl or methylenedioxy are new.

The compounds of the general Formula VI are prepared by reacting a racemic or optically active compounds of the general Formula VII

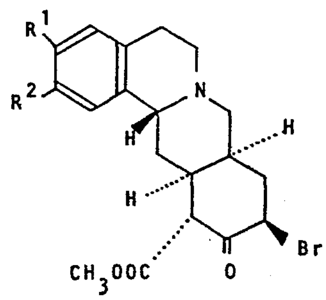

(VII)

./...

EP 0 091 185 A2

with trimethoxy-benzoic acid or a derivative - preferably a salt - thereof and isolating the compound of the general Formula VI thus obtained and if desired converting a base produced into a salt thereof or liberating the free base from a salt.

0091185

BERBANE DERIVATIVES

This invention relates to new berbane derivatives, a process for the preparation thereof and pharmaceutical compositions containing the same.

According to an aspect of the present invention there is provided a process for the preparation of racemic and optically active berbane derivatives of the general Formula VI

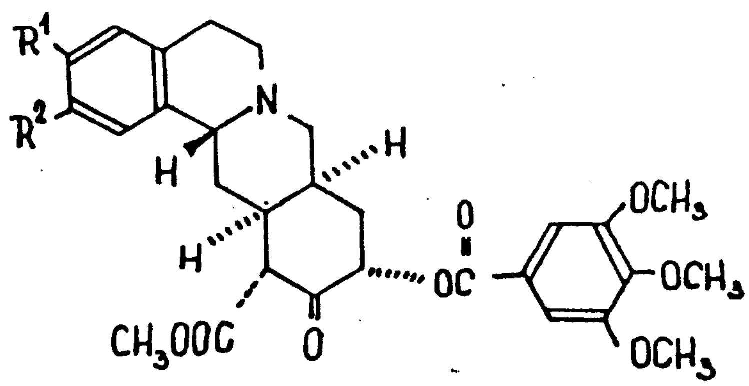

(VI)

wherein

$R^1$ and $R^2$ each stands for an alkyl group having 1-4 carbon atoms or

$R^1$ and $R^2$ together form a methylenedioxy group.

The compounds of the general Formula VI

- wherein

$R^1$ and $R^2$ stand for $C_{2-4}$ alkyl or form a methylene dioxy group

are new and possess valuable pharmaceutical properties,

particularly hypotensive effect.

According to a further feature of the present invention there are provided new racemic or optically active berbane derivatives of the general Formula VI and salts thereof.

According to a still further feature of the present invention there are provided pharmaceutical compositions comprising as active ingredient a racemic or optically active berbane derivative of the general Formula VI or a salt thereof in admixture with one or more inert non-toxic pharmaceutical carriers.

According to the process of the present invention the racemic or optically active berbane derivatives of the general Formula VI and salts thereof are prepared by reacting a racemic or optically active compound of the general Formula VII

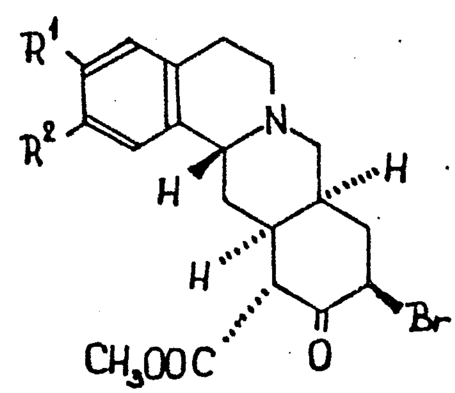

(VII)

with a derivative of trimethoxy benzoic acid - preferably a salt thereof - and isolating the product thus

obtained, and if desired converting a free base obtained into a salt or liberating the base from a salt initially obtained.

The details of the process of the present invention are shown in connection with the preparation of the methylenedioxy derivative of the Formula I.

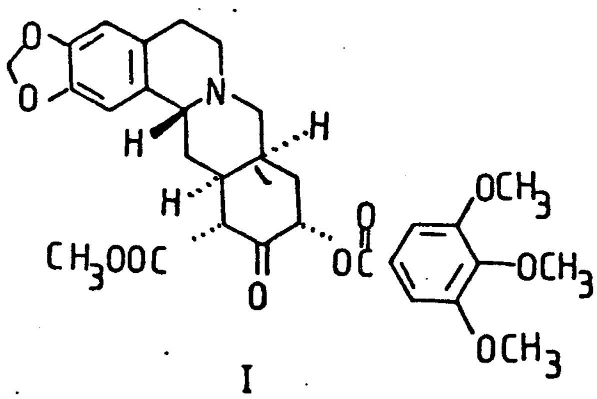

I

(I)

The racemic compound [(±),I] is prepared by reacting the known (±)-methyl-15-bromor-7,8-methylenedioxy-14-oxo-epiallo-berbane-13-carboxylate [Chem. Ber. 109 (1976)] with a salt of trimethoxy benzoic acid. The base thus obtained can be converted into salts thereof formed with pharmaceutically acceptable acids (e.g. hydrochloric acid, sulfuric acid, tartaric acid, citric acid etc).

The optically active antipodes are prepared by resolving the racemic ketone of the Formula II

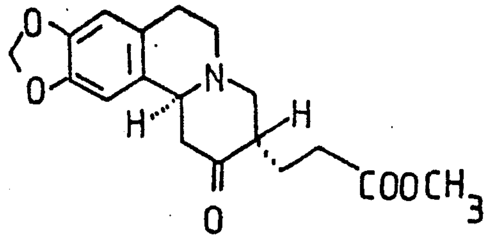

II

(II)

[Acta Chim. Hung. 100, 19 (1979)] into the optically active antipodes by treatment with + (+)-D-tartaric acid in ethyl acetate and subjecting the dextrorotatory enantiomer thus obtained to the following sequence of reactions.

The ketone [(±),II] is condensed with methoxycarbonyl-methyl-phosphonic acid diethylester, the isomeric mixture thus obtained is subjected to Dieckmann cyclisation and subsequently to reduction whereby the allo type (III)

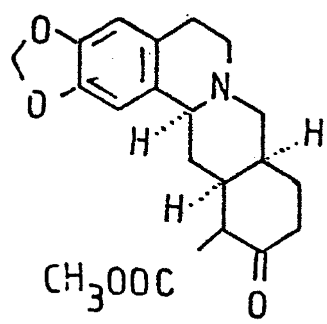

III

(III)

and epiallo type (IV)

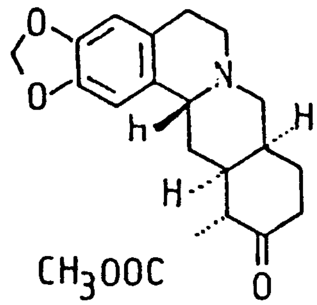

(IV)

IV

dextrorotatory products are obtained. The epiallo compound (IV)' is brominated to yield the dextrorotatory bromo derivative [(±), V]

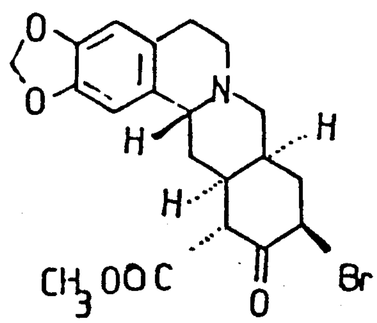

(V)

V

which is then reacted with a salt of trimethoxy benzoic acid to give the dextrorotatory compound [(±),I].

The <u>allo</u> compound (III) is oxidized into an iminium salt which is reduced with $Zn^{+}$ in acidic

medium to yield the laevorotatory compound (VI) being the enantiomer of compound IV. This compound is brominated and the [(-),V] compound thus obtained is reacted with a salt of trimethoxy benzoic acid to yield laevorotatory compound [(-),I] being the enantiomeric form of [(+),I].

The above method enables the preparation of both - dextrorotatory and laevorotatory - enantiomers of the desired product from a single and common optically active enantiomer [(+), II] without any intermediate racemization and resolution. The same results can be achieved by carrying out the above sequence of reaction with the laevorotatory compound [(-), II ] obtained after the resolution of [($\pm$), II].

The absolute configurations shown on the Formulae are stipulated ones.

The toxicity of the product is so low that it can be neglected. The pharmacological activity was determined by the following test. Derivatives containing two alkoxy groups in the place of the methylene dioxy group exhibit similar activity.

Pharmacological tests

The berbane derivatives of the general Formula I [(+)(-)] possess particularly significant and lasting hypotensive effect. The tests were carried out on CFY strain male rats weighing 250-300 g. The systolic blood pressure was measured indirectly on the tails of the animals pressed down with the aid of a cuff. When the pressure of the pressing cuff attached to a mercury manometer decreased just under the systolic pressure, the sensing head of the measuring apparatus (VP 101 microphone adapted for this purpose and placed on the tails of the rats) signalled the appearence of arterial pulsation both in acustical and visual way (oscilloscope). The systolic blood pressure was determined by observing simultaneously the manometer and the out-signal. When administering the racemic compound intraperitoneally in doses of 10 and 20 mg/kg, a significant hypotensive effect was measured 3-6 hours after the administration. The effect reached its maximum in the sixth hour, thereafter decreased gradually and the effect could be still detected in the 48th hour. The characteristics of the hypotensive effect of reserpine used as control are substantially the same as those of the racemic compound; hypotension of approximately the same intensity and duration can be achieved by administering 2,5 mg/kg of reserpine and

20 mg/kg of the racemic compound, respectively.

The optically active enantiomers of the compound also decrease systolic blood pressure; this effect of the enantiomers does not differ significantly. The optically active enantiomers were administered in intraperitoneal doses of 20 mg/kg to male rats weighing 300 and having hypertension. In the sixth hour following administration of the test compound (at the maximum of the effect) the dextrotatory enantiomer [(+), I] induced a blood-pressure decrease of 15,6 Hgmm (average, n = 6) while the laevorotatory enantiomer [(-), I] caused a blood-pressure decrease of 14,8 Hgmm (average, n =6).

Contrary to reserpine, the racemic compound [(±), I] exhibits its hypotensive effect without inhibiting the functions of central nervous system, as shown by a comparison of the effects exerted on coordination. These tests were carried out on CFLP strain female mice weighing 20-25 g by the rotating rod method [A. Kovach: Testing methods of experimental medical sciences - A kisérletes orvostudomány vizsgáló módszerei - Akadémia kiadó, Budapest, VI. 232-233, 1962)]. In the sixth hour following administration (at the period of time of the maximum of hypotensive effect) the racemic compound did not exhibit any effect on

coordination, while reserpine roduced coordination to a very significatn extent (Table 2).

**Table 1**

**Effect of a berbane derivative of the Formula I (racemic compound) and reserpine on the systolic blood-pressure of non-anaesthetized rats**

| Test compound and dosage thereof | n | Blood pressure (Hgmm; $\bar{x} \pm$ S.E.) Control | 3rd | 6th | 12th | 18th | 24th | 48th hour |
|---|---|---|---|---|---|---|---|---|
| BERBANE DERIVATIVE (SC-T 40) | | | | | | | | |
| 10 mg/kg i.p. | 5 | 115 ± 2 | 110 ± 2 (-4,23 %) | xxx 99 ± 3 (-13,88%) | xxx 101 ± 2 (-12,14 %) | xxx 104 ± 2 (-9,53%) | xxx 104 ± 4 (-9,64 %) | xxx 104 ± 2 (-9,53%) |
| 20 mg/kg i.p. | 5 | 116 ± 2 | xxx 107 ± 2 (-7,98 %) | xx 93 ± 6 (-19,53 %) | xxx 96 ± 2 (-17,11 %) | xxx 101 ± 3 (-12,76 %) | xxx 105 ± 2 (-9,42 %) | xxx 111 ± 1 (-4,23%) |
| RESERPINE | | | | | | | | |
| 2,5 mg/kg i.p. | 5 | 117 ± 2 | xxx 104 ± 2 (-11,11 %) | xxx 95 ± 2 (-18,8 %) | xxx 106 ± 2 (-9,4 %) | xxx 102 ± 1 (-12,82 %) | xxx 105 ± 3 (-10,26 %) | xxx 109 ± 2 (-6,48%) |
| 5,0 mg/kg i.p. | 5 | 118 ± 2 | xxx 100 ± 5 (-15,25 %) | xx 95 ± 7 (-19,49 %) | xxx 102 ± 1 (-13,56 %) | xxx 108 ± 2 (-8,47 %) | xxx 106 ± 3 (-10,17 %) | xxx 111 ± 2 (-5.93%) |

The statistical significance related to the starting (control) value is as follows:

xx p 0,025 xxx p 0,005

## Table 2

Effects of a berbane derivative of the Formula I (racemic compound) and reserpine on the central nerval system

(Measurement of coordination on mice by the rotating rod method)

| Test compound and dosage thereof | Time of measurement, after administration of the test compound[x] | Time for which mice stayed on the rotating rod (average, n = 10) | p | Activity quotient[xx] |
|---|---|---|---|---|
| Control | | 98 ± 9,3 | | |
| BERBANE DERIVATIVE | | | | |
| (SC-T 40) | | | 0,35 | |
| 10 mg/kg i.p. | 6th hour | 102 ± 12,0 | 0,40 | 1,04 |
| Control | | 117 ± 3,0 | | |
| RESERPINE | | | | |
| 5 mg/kg i.p. | 6th hour | 9 ± 3,3 | 0,0005 | 0,077 |

[x] The time of measurement was determined on the basis of the interval required to achieve maximal hypotensive effect.

[xx] Quotient of values obtained for the treated and control animals.

The pharmaceutical compositions according to the present invention comprising a racemic or optically active berbane derivative of the general Formula VI or a salt thereof are prepared by methods known per se.

Further details of the present invention are to be found in the following Examples without limiting the scope of the invention to the Examples.

Example 1

(+)-2-oxo-3a-(ß-methoxycarbonylethyl)-9,10-
-methylenedioxy-1,2,3,4,6,7-hexahydro-
-11αH-benzo(a)quinolizine (1)

10 g of racemic benzo(a)quinolizine (II) are dissolved in 300 ml of ethyl acetate whereupon to the hot solution thus obtained a solution of 4,789 g of (+)-D-tartaric acid and 30 ml of methanol is added. The solution formed is allowed to crystallize for 24 hours, the precipitated crystals are filtered off and washed with ethyl acetate. Thus 6,72 g of the title compound are obtained, yield 91 %. $[\alpha]_D = 76,8^o$ (methanol). Mp.: 155-157 $^oC$.

The crystals are dissolved in 300 ml of water, the solution is made alkaline with a 5 % sodium hydrogen carbonate solution and extracted with dichloro methane. The organic layer is dried over magnesium

sulfate and evaporated to dryness. Thus 4,0 g of the product are obtained, yield 80 %, $[\alpha]_D^{25}$ =+134,8° (methylene chloride).

Mp.: 116-118 °C (methanol).

The methanol mother lye is evaporated to dryness, the residue is dissolved in 300 ml of water, made alkaline with 5 % sodium hydrogen carbonate solution and absorbed with methylene chloride. The organic layer is evaporated and the residue is crystallized from methanol. Thus 4,0 g of the product are obtained, yield 80 %.

Mp.: 111-113 °C, $[\alpha]_D^{25}$ = -136,6° (methylene chloride).

Example 2

(+)-2-methoxycarbonylmethylene-3α-(ß-methoxycarbonylethyl)-9,10-methylenedioxy-1,2,3,4,6,7--hexahydro-11bα-benzo(a)quinolizine (2)

(-)-2-methoxycarbonylmethylene-3α-(ß-methoxycarbonylethyl)-9,10-methylenedioxy-1,2,3,4,6,7--hexanydro-11bαH-benzo(a)quinolizine (3)

A solution of 1,02 g (3,07 millimoles) of (+)-2-oxo-3α-(ß-methoxycarbonylethyl)-9,10-methylenedioxy-1,2,3,4,6,7-hexahydro-11bαH-benzo(a)quinolizine and 5 ml of dimethyl formamide is poured into a solution of 0,86 g (7,6 millimoles) of potassium tertiary butylate and 2,0 g (9,43 millimoles) of methoxycarbonyl--methyl-phosphonic acid diethylester and 3 ml of dimethyl formamide. The reaction mixture is allowed to stand at room temperature for 2 days whereupon it is poured into 100 ml of icecold water and extracted three times with 30 ml ether each. The ether phases are united, evaporated and the crude mixture thus obtained is used in the next step.

A sample of the product is subjected to chromatography on a silicagel plate ($PF_{254-366}$; developing agent: 14:3 benzene-methanol mixture eluent: a mixture of acetone, methylene chloride and methanol.

(2) upper phase: oil $[\alpha]_D^{25} = 51^o$ (methylene chloride)

(3) lower phase: mp.: 90 $^oC$ $[\alpha]_D^{25} = 15^o$ (methylene chloride)

Example 3

(+)-methyl-7,8-methylenedioxy-14-oxo-allo--berbane-13-carboxylate; (4), [(+),III]

(+)-Methyl-7,8-methylenedioxy-14-oxo-epiallo--berbane-13-carboxylate, (5), [(+), IV]

The crude mixture of the dimethyl esters obtained above [(2); 1,2 g, 3,0 millimoles] is heated to boiling with 0,865 g (7,73 moles) of potassium tertiary butylate in 11 ml of anhydrous benzene for 20 minutes, whereupon the solution is evaporated to dryness and a sodium methalyte solution [prepared from 20 ml of methanol and 0,46 g (20 millimoles) of sodium] is added. The mixture is allowed to stand for 2 hours

and hydrogenated in the presence of 1 g of a palladium-charcoal catalyst. When no more hydrogen is taken up, to the reaction mixture 1,66 g (27,7 millimoles) of glacial acetic acid are added, the solution is evaporated to dryness, the residue is treated with water and extracted three times with 20 ml of dichloro methane each. The organic extracts are united, dried over magnesium sulfate and evaporated to dryness. The residue is separated on KG-$PF_{254-366}$ plates (benzene: methanol = = 14:2).

Upper phase: (4): 324 mg, yield 30 %.
Mp.: 135-138 °C (methanol)
$[\alpha]_D^{20}$ = +98° (methylene chloride)

Lower phase: (5): 307 mg, yield 28 %
Mp.: 160-161 °C
[α] = +126° (methylene chloride).

Example 4

(-)-Methyl-7,8-methylenedioxy-14-oxo-epiallo-berbane-13-carboxylate (7), [(+), V]

a.) 7,8-methylenedioxy-13-methoxy-carbonyl-14-oxo-1,2-berbanium-perchlorate (6)

A mixture of 0,5 g (1,4 millimoles) of (+)-methyl-7,8-methylenedioxy-14-oxo-allo-berbane-13-carboxylate (5), 5 ml of glacial acetic acid and 0,7 g (2,2 millimoles) of mercury (II) acetate is stirred

at 100 °C for 3 hours. The reaction mixture is poures into icecold water, made alkaline with ammonium hydroxide (pH = 9) and extracted three times with 20 ml of dichloro methane each. The organic extracts are united, dried and evaporated to dryness. The residue is dissolved in 2 ml of methanol and 0,5 ml of 70 % perchloric acid are added under cooling. The precipitated crystals are filtered and wasted with ether. Thus 0,5 g of (6) are obtained, yield 78,7 %.

Mp.: 218-220 °C.

IR (KBr): 1730, 1700 (CO, $COOCH_3$) 1650 (C=N) 1595 (aromatic), 1110-1090 (perchlorate)

Ms (m/e): 355 (100), 354 (55), 340 (13), 324 (40), 322 (40), 310 (33), 299 (20), 298 (40), 297 (38), 296 (58), 295 (43), 294 (17), 282 (9), 268 (18), 267 (42), 266 (52), 254 (13), 252 (13), 240 (77), 239 (57), 226 (117), 206 (30), 189 (42), 175 (25), 160 (18), 85 (62), 63 (97), 44 (200).

b.) The berbanium perchlorate (1,1 millimoles) thus obtained (6) is stirred in a mixture of 20 ml acetone, 4 ml of water and 6 ml of 10 % hydrochloric acid, whereupon a catalytic amount of ferric(III) chloride and mercury(II) chloride are added and thereafter 2,5 g of zinc powder are added in small portions within about 2,5 hours. The unreacted zinc powder removed by filtration,

the filtrate is evaporated to about one fifth of its volume, the residue is made alkaline with ammonium hydroxide to pH = 9 and extracted three times with 20 ml of dichloro methane each. The dichloro methane phase is dried and evaporated to dryness. The residue is separated by preparative layer chromatography or on a column (pressure 1,7 atm) in a 14 : 1 benzene/methanol mixture.

(4) upper phase: 80 mg (yield 20 %) starting material.
(7) lower phase: 280 mg (yield 56 %), mp.: 160 °C
$[\alpha]_D^{20}$ = 117 °C (methylene chloride).

Example 5

(+)-Methyl-15α-bromor-7,8-methylenedioxy--14-oxo-*epiallo*-berbane-13-carboxylate--hydrobromide (8) [(+), V]

1,0 g (2,8 millimoles) of methyl-(+)-7,8--methylenedioxy-14-oxo-*epiallo*-berbane-13-carboxylate (5) are admixed with 10 ml of glacial acetic acid under stirring at 5-7 °C whereupon a solution of 0,466 g (2,8 millimoles) of bromine and 3 ml glacial acetic acid is added. The reaction mixture is stirred at room temperature for 3 hours and at 95-100 °C for 5 hours, whereupon it is cooled and poured into 50 ml of ether. The precipitated crystals are filtered off and washed with ether. Thus 1,1 g of the title compound are

obtained.

Yield: 76 %.

Mp.: 250-48 °C.

Example 6

(-)-Methyl-15α-bromo-7,8-methylenedioxy--14-oxo-*epiallo*-berbane-13-carboxylate--hydrobromide (9) [(-), V]

(-)-Methyl-7,8-methylenedioxy-4-oxo-*epiallo*--berbane-13-carboxylate (7) is brominated according to the method described in Example 5. The title compound is obtained with a yield of 70 %.

Mp.: 218-227 °C.

Example 7

(+)-Methyl-7,8-methylenedioxy-15-(3,4,5--trimethoxy-benzoyloxy)-14-oxo-*epiallo*--berbane-13-carboxylate [(+), I]

A mixture of 1,0 g (1,93 millimoles) of (+)-methyl-15-bromo-7,8-methylenedioxy-14-oxo-*epiallo*--berbane-13-carboxylate-hydrobromide, (8), 10 ml of anhydrous dimethyl formamide and 1,0 g (3,99 millimoles) of potassium-3,4,5-trimethoxy-benzoate is stirred at 100 °C for an hour. The reaction mixture is cooled, poured into 50 ml of icecold water and made alkaline with a 5 % sodium hydrogen carbonate solution (pH = 8). The precipitated crystals are filtered off and recrystallized from methanol. Thus 0,47 g of the title

compound are obtained, yield 44,3 %.

Mp.: 184-186 °C (methanol)

$[\alpha]_D^{25}$ = 24,5° (methylene chloride).

On acidifying the methanol mother-lye with methanol containing hydrochloric acid, the crystalline hydrochloride is obtained with a yield of 95 %.

Example 8

(-)-Methyl-7,8-methylenedioxy-15-(3,4,5--trimethoxy-benzoyloxy)-14-oxo-*epiallo*--berbane-13-carboxylate [(-), I]

The title compound is obtained from the (-)-bromoketo-ester (9) according to the process described in Example 7 with a yield of 41 %.

$[\alpha]_D^{25}$ = -25° (methylene chloride),

Mp.: 186-191 °C.

Example 9

Racemic methyl-7,8-methylenedioxy-15-(3,4,5--trimethoxy-benzoyloxy)-14-oxo-*epiallo*--berbane-13-carboxylate [(±), I]

A mixture of 1,0 g (1,93 millimoles) of racemic methyl-15-bromo-7,8-methylenedioxy-14-oxo--*epiallo*-berbane-13-carboxylate-hydrobromide, 10 ml of anhydrous dimethyl formamide and 1,0 g (3,99 millimoles) of potassium-3,4,5-trimethoxy-benzoate is stirred at 100 °C for an hour. The reaction mixture

is cooled, poured into 50 ml of icecold water and made alkaline with an 5 % sodium hydrogen carbonate solution (pH = 8). The precipitated product is filtered off, washed with water and recrystallized from methanol.

Thus 0,87 g of the title compound are obtained, yield 82 %.

Mp.: 186 °C (methanol).

Analysis: for the Formula $C_{30}H_{33}NO_{10}$ (567,57)

calculated: C % 63,48; H % 5,86; N % 2,47;

C % 63,31; H % 5,71; N % 2,70.

IR (KBr): 2750-2800 (Bohlmann-band), 1740, 1720, 1715 (OTMB, $COOCH_3$, CO).
1595 $cm^{-1}$ (aromatic).

NMR ($C_6D_6$+DMSO): 7,56 (2H, s, $C_2$,-H,$C_6$-H), 6,68, 6,56 (2H, s, $C_6$-H, $C_9$-H), 5,91 (2H, s, $OCH_2O$), 5,57 (1H, m, $J_{ae}$ = 6 Hz, $J_{aa}$ = 12 Hz, C15ax-H), 3,89, 3,87, 3,84 (12H, s, $COOCH_3$ $OCH_3$).

MS (m/e) 567 (29), 566 (14,6), 552 (4,6), 536 (4), 535 (3,5), 534 (1,9), 5 (19), 416 (6,3), 415 (11), 374 (8,5), 373 (32,7), 371 (4), 370 (3,7), 356 (59,8), 355 (88,5), 354 (31), 340 (27), 324 (19), 314 (17), 312 (13), 297 (48), 296 (52), 266 (13,5), 242 (17), 240 (23), 228 (15,4), 226 (35), 216 (52), 214 (21), 212 (100), 202 (15), 197 (46),

195 (56), 190 (21), 189 (94), 188 (13,5), 187 (13,5), 175 (48), 174 (33), 142 (15), 77 (17), 45 (15), 44 (38), 41 (17).

The precipitated product is suspended in 5 ml of methanol and thereafter methanol containing hydrochloric acid is added. The hydrochloride is obtained with a yield of 95 %.

CLAIMS

1. Racemic or optically active berbane derivatives of the general Formula VI

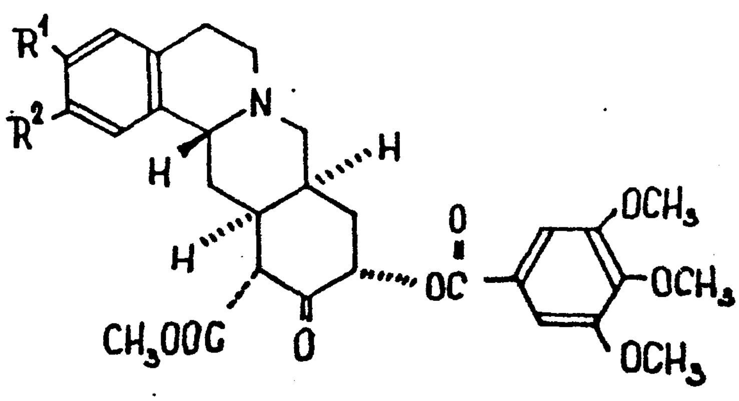

(VI)

wherein

$R^1$ and $R^2$ each stands for an alkyl group having 2-4 carbon atoms or

$R^1$ and $R^2$ together form a methylenedioxy group

and pharmaceutically acceptable salts thereof.

2. (±)-Methyl-7,8-methylenedioxy-15-(3,4,5--trimethoxy-benzoyloxy)-14-oxo-*epiallo*-berbane-carboxylate.

3. (-)-Methyl-7,8-methylenedioxy-15--(3,4,5-trimethoxy-benzoyloxy)-14-oxo-*epiallo*-berbane--carboxylate.

4. (+)-Methyl-7,8-methylenedioxy-15--(3,4,5-trimethoxy-benzoyloxy)-14-oxo-*epiallo*-berbane--carboxylate.

5. Pharmaceutical composition comprising as active ingredient a compound of formula (VI) (as defined in claim 1 but wherein $R^1$ and $R^2$ stand for $C_{1-4}$ alkyl or form together a methylenedioxy group) or a pharmaceutically acceptable salt thereof in admixture with one or more pharmaceutical carriers.

6. Process for the preparation of a compound as claimed in claim 1 which comprises reacting a racemic or optically active compound of the general formula VII

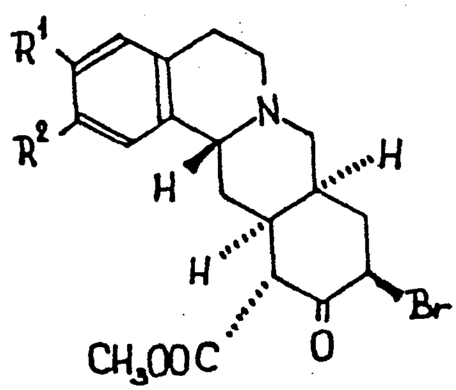

(VII)

with trimethoxy-benzoic acid or a salt or other derivative thereof and isolating the compound of the general Formula VI thus obtained, and thereafter if desired converting a base of Formula VI obtained into a pharmaceutically acceptable salt thereof of liberating the free base from a salt of Formula VI obtained.

7. Process according to Claim 6 which comprises using potassium trimethoxy benzoate.

8. Process for the preparation of a pharmaceutical composition according to Claim 5 which comprises admixing the active ingredient as defined in Claim 5

with one or more pharmaceutical carriers.